# EUROPEAN PATENT SPECIFICATION

(11) **EP 1 005 557 B1**
(45) Date of publication and mention of the grant of the patent: **28.02.2007**
(21) Application number: 98938339.3
(22) Date of filing: 05.08.1998
(51) Int. Cl.: C12N 15/60, C12N 9/88, C12N 15/70, C12N 1/21

(54) **HIGH EXPRESSION MODULES CONTAINING TWO OR MORE TANDEM COPIES OF A METHIONINASE ENCODING SEQUENCE**
HOCHEXPRIMIERENDE MODULE MIT ZWEI ODER MEHREREN TANDEMKOPIEN EINER FÜR METHIONINASE KODIERENDEN SEQUENZ
MODULES A HAUTE EXPRESSION CONTENANT AU MOINS DEUX COPIES TANDEM D'UNE SEQUENCE CODANT LA METHIONINASE

(30) Priority: 08.08.1997 US 908860
(43) Date of publication of application: 07.06.2000
(73) Proprietor: ANTICANCER, INC., San Diego, CA 92111 (US)
(72) Inventor: TAN, Yuying, San Diego, CA 92111 (US)
(74) Representative: Sexton, Jane Helen
(86) International application number: PCT/US1998/016220
(87) International publication number: WO 1999/007858

(56) References cited:
- WO-A-94/11535
- WO-A-96/40284
- DATABASE WPI Section Ch, Week 9738 Derwent Publications Ltd., London, GB; Class B04, AN 97-409889 XP002092005 & JP 09 182592 A (SHIONOGI & CO LTD) , 15 July 1997
- HIROYUKI INOUE ET AL.: "Structural analysis of the L-methionine gamma-lyase gene from Pseudomonas putida" JOURNAL OF BIOCHEMISTRY., vol. 117, 1995, pages 1120-1125, XP002092004 TOKYO JP
- TAN Y ET AL: "Overexpression and large-scale production of recombinant l -methionine-alpha-deamino-gamma-mercaptome thane-lyase for novel anticancer therapy" PROTEIN EXPRESSION AND PURIFICATION, vol. 9, no. 2, March 1997, pages 233-245, XP002082381

## Description

### Technical Field

The present invention relates to methioninase expression systems containing two or more tandem nucleotide sequences that encode methioninase and to methods to produce recombinant methioninase from them. This system of production results in high amounts of methioninase activity. The methioninase thus produced is useful in antimethionine and antihomocysteine therapy.

### Background

Therapeutic drug-based treatment of cancer is directed at the use of medicinals which selectively inhibit or kill the cancer cells while not harming normal tissue function beyond acceptable amounts. The difficulty with conventional chemotherapy has been the toxicity of therapeutic drugs for normal tissue.

Many tumors have been shown to have absolute requirement for methionine in a variety of cell types and evaluated tumor tissues, including tumors of the colon, breast prostate, ovary, kidney, larynx melanoma, sarcoma, lung, brain, stomach and bladder as well as leukemias and lymphomas. Methionine dependence has been defined as an inability of tumors to grow when methionine is replaced by homocysteine in the growth medium. See, for example, *Chello et al., Cancer Res,* 33:1898-1904, 1973; and Hoffman, *Anticancer Res,* 5:1-30, 1985.

Methionine depletion has been shown to synchronize selectively methionine-dependent tumor cells into late S/G₂ phase of the cell cycle. Hoffman et al, *Proc Natl Acad Sci USA,* 77:7306-7310, 1980. Using antimethionine chemotherapy which is methionine deprivation, followed by depletion of methionine and coupled with exposure to an antimitotic agent, tumor cells have been selectively eliminated from cocultures of normal and tumor cells, resulting in cultures of normal cells proliferating vigorously. Stern *et al., J Natl Cancer Inst,* 76:629-639, 1986.

In order for methionine-dependent chemotherapy to be conducted *in vivo,* it is necessary to have a means to effectively deplete serum of circulating methionine. Methionine depletion methods have not been described that reduce circulating methionine levels *in vivo* in a manner sufficient to be effective in antitumor therapies.

Methioninase, an enzyme which degrades methionine, has been purified from a variety of bacterial sources, and has been reported to slow the rate of tumor cell proliferation *in vitro.* Kreis *et al., Cancer Res,* 33:1862-1865, and 1866-1869, 1973; Tanaka *et al., FEBS Letters,* 66:307-311 1976; Ito *et al., J Biochem* 79:1263-1272, 1976; and Nakayama *et al., Agric Biol Chem* 48:2367-2369, 1984.

Kreis *et al., Cancer Res* 33:1866-1869, 1973, have described the use of highly impure methioninase preparations isolated from *Clostridium sporogenes* at 1150 units/kg/day to inhibit growth of carcinosarcoma cells implanted in a mouse model. Although the enzyme apparently reduced primary tumor cell growth, it was not reported to reduce the T/C (treated versus control) ratio of tumor diameter below 50%, and was not reported to have any effect on metastasis. The authors also indicated that tumor specificity of the methioninase cannot be expected without other unspecified interventions, and further do not comment on the possibility that endotoxin, or other components of the impure preparation, were responsible for the effects observed. The only toxicity studies reported were absence of animal body weight loss after the duration of the treatment, and negative gross examination for toxicity. Further, the authors report that the enzyme had a serum half life of 4 hours. *Kreis et al., Cancer Res* 33:1866-1869, 1973, further reported the use of a methionine-free diet as a means to deplete methionine as an antitumor therapy, but the diet did not slow tumor growth as effectively as the use even of an impure preparation of methioninase and resulted in the undesirable side effect of continuous loss of weight of the animal.

The parent applications herein disclose effective chemotherapy of tumors directed at reducing the amount of methionine as to provide a beneficial antitumor effect without deleterious injury using methioninase. The present invention improves the disclosed therapeutic and diagnostic methods and composition by providing a method to produce commercially viable quantities of highly pure recombinant methioninase, using expression systems containing at least two copies of the methioninase gene.

### Disclosure of the Invention

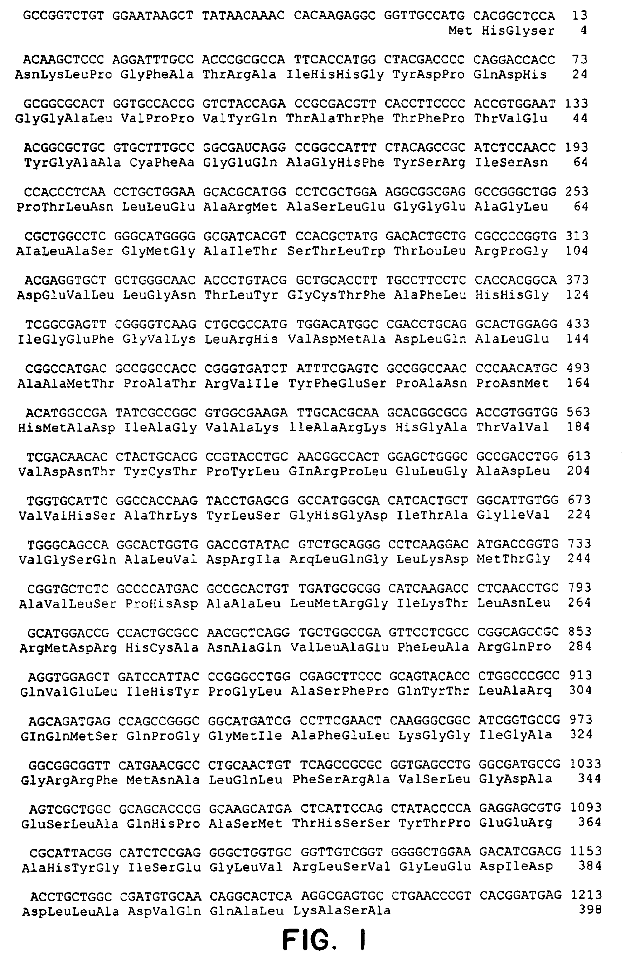

The present invention is based, in part, on the generation of methioninase expression systems containing two or more tandem copies of methioninase encoding nucleotide sequences. In particular, the present invention provides an expression vector capable of high level production of methioninase, comprising two or more tandem copies of a nucleotide sequence encoding methioninase operably linked to a promoter
wherein said nucleotide sequence is selected from the group consisting of:
a) a nucleotide sequence that encodes a protein having the amino acid sequence shown in Figure 1,
b) a nucleotide sequence as shown as encoding protein in Figure 1, and
c) a nucleotide sequence that encodes a methioninase which is encoded by a nucleotide sequence that hybridizes to the complementary strand of a nucleotide sequence encoding protein as shown in Figure 1 under high stringency conditions.

The expression vector of the present invention can produce recombinant methioninase in an appropriate host cell, such as *E*. *coli,* at levels ranging from about 40 to 75% of total cellular protein.

In a preferred embodiment methioninase expression vectors containing two or more tandem copies of the *P. putida* methioninase gene operably linked to a T7 RNA polymerase promoter are described. These systems have been used to produce recombinant methioninase at about 1 to 4 gram/liter, with an activity of about 6.4 to about 12.4 units/ml, and a specific activity of about 3.8 to about 10.2 units/mg before purification, using appropriate incubation conditions and purification methods.

The invention further provides methods of producing recombinant methioninase using cells containing the methioninase expression vector of the present invention.

Substantially pure recombinant methioninase produced using cells containing the methioninase expression vectors of the present invention is useful in compositions for diagnostic and therapeutic use, particularly in methods for inhibiting tumor cell growth to lower homocysteine levels in patients to reduce the risk of, and to treat, cardiovascular diseases, obesity and negative symptomologies of aging, as well as to deplete methionine for tumor diagnosis and imaging.

The recombinant methioninase may be provided in chemically modified forms, for example by coupling to polymers such as polyethylene glycol (PEG).

### Brief Description of the Drawings

Figure 1 provides the nucleotide (and corresponding amino acid sequence) of a methioninase encoding DNA molecule isolated from *P. putida.*
Figure 2 provides an outline of the purification steps used to obtain highly pure, endotoxin free methioninase.
Figure 3 is a diagram of a one-copy and a two-copy methioninase expression system.
Figure 4 compares methioninase expression levels as a function of time using single and two-copy expression systems.

The drawings are not necessarily to scale, and certain features of the invention may be exaggerated in scale and shown in schematic form in the interest of clarity and conciseness.

### Modes of Carrying out the Invention

As used herein, a "multicopy" or "multiple" expression system refers to a nucleic acid molecule that contains one or more expression control elements that direct the transcription and translation of two or more tandem copies of a nucleotide sequence that encodes methioninase. Preferably, the expression system will contain from two to four tandem copies of the methioninase encoding sequences which may be the same or different. If desired, the nucleotide sequences can be modified from those found in nature to contain silent mutations to provide codons preferred by *E. coli.* Such codons are known in the art -- see, e.g., U.S. Patent Nos. 4,356,270 and 4,571,421 to Genentech.

The resulting recombinant methioninase produced represents from about 40-75% of total cellular protein, preferably more than 50% of total cellular protein. The preferred expression control element is the T7 RNA polymerase promoter. Other examples of RNA polymerase promoters include, but are not limited to, the Tac and Trc promoters.

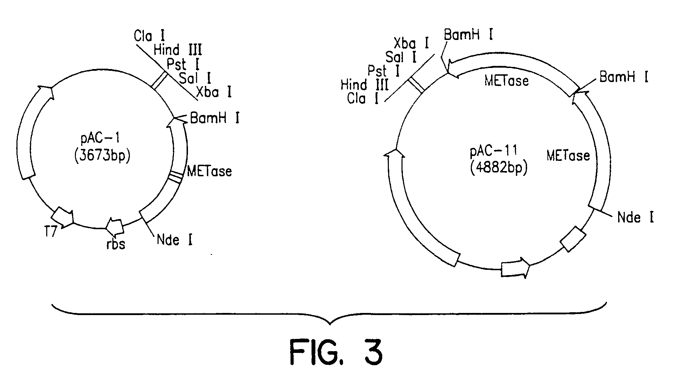

As used herein, a nucleotide sequence is said to "encode" methioninase when the transcription and translation of the sequence results in the production of a protein having methioninase activity. Copies of methioninase encoding nucleotide sequences are "tandemly" oriented when they are provided as direct repeats of the same or different methioninase-encoding sequence. Figures 3 provides a diagram of a multiple expression system that contains two tandem copies of the *P. putida* methioninase gene.

L-Methioninase (L-methionine-α-deamino-γ-mercaptomethane-lyase or methioninase) is an enzyme that degrades methionine by deamination and dethiomethylation, to produce α-ketobutyrate. One method to measure methioninase activity is to determine the amount of α-ketobutyrate formed. One unit (U) of methioninase is defined as an amount of enzyme that produces 1 micromole of α-ketobutyrate per minute from methionine under the standard assay conditions described by Ito *et al., J Biochem,* 79:1263-1272, 1976; and Soda, *Analyt Biochem* 25:228-235, 1968.

The methioninase-encoding nucleotide sequence may be unaltered as, obtained from an organism that naturally produces this enzyme or may be modified to result in one or more amino acid substitutions, deletions or additions.

The methioninase-encoding nucleic acid molecule, whether altered or unaltered, can be derived from any organism that naturally produces methioninase. The preferred source of the methioninase-encoding nucleic acid molecule is *Pseudomonas putida.* Example 1 discloses the isolation and sequencing of a methioninase-encoding nucleic acid molecule from *P. putida* (See Figure 1). Other preferred sources include, but are not limited to, *Trichomonas vaginalis, Nippostrongylus brasiliensis,* and *Fusobacterium* sp.

The multicopy expression systems can be prepared using art known methods, such as restriction digestion followed by ligation.

If desired, the methioninase encoding sequences may be altered so as to facilitate purification of the resulting protein, for example, by adding a polyhistidine stretch at either the amino or carboxy terminus. Ni⁺⁺ sepharose can then be used to purify the resulting fusion protein.

The present invention further provides vectors containing one or more of the multicopy expression systems of the present invention. Vectors are DNA molecules that can be introduced into host cells and may be capable of autonomous replication within a host. Vectors may thus contain an episomal origin of replication derived from a naturally occurring plasmid, a genomic origin of replication, or may be derived from a viral genome. The choice of the vector to which an expression system of the present invention is inserted depends on the functional properties desired, e.g., presence of a suitable marker, and the host cell to be modified.

In one preferred embodiment, the vector includes a procaryotic replicon, such as the ColE1 replicon, as well as a selectable marker such as a drug resistance.

Eucaryotic expression vectors can also be used, are well known in the art and arc available from several commercial sources. Typical such vectors are pSVL and pKSV-10 (Pharmacia), pBPV-1/pML2d (International Biotechnologies, Inc.), pTDT1 (ATCC, #31255), and the vector pCDM8 described herein. High level expression vectors can further be generated using insect cell expression systems such as a bacculovirus based vector system.

The host cells for methioninase production can be either procaryotic or eucaryotic. A preferred procaryotic host is *E. coli.* In the Examples that follow, the DH5α and BL21(DE3) strains of *E*. *coli* were used.

Preferred eucaryotic host cells include insect cells, yeast cells and mammalian cells, preferably insect cells such as SP6 and vertebrate cells such as those from a mouse, rat, monkey or human fibroblastic cell line. Other preferred eucaryotic host cells include Chinese hamster ovary (CHO) cells available from the ATCC as CCL61, NIH Swiss mouse embryo cells NIH/3T3 available from the ATCC as CRL 1658, baby hamster kidney cells (BHK), and the like eucaryotic tissue culture cell lines.

Transformation of an appropriate host with a multicopy methioninase expression system of the present invention is accomplished by well known methods that typically depend on the type of host and vector used. Transformation of procaryotic host cells is preferably by electroporation or salt treatment; for example, see Cohen *et al., Proc Natl Acad Sci USA* 69:2110, 1972; and Maniatis *et al., Molecular Cloning. A Laboratory Mammal,* Cold Spring Harbor Laboratory, Cold Spring Harbor, NY (1982). Transformation of eucaryotic cells is preferably by electroporation or the use of a cationic lipid, for example, see Graham *et al., Virol* 52:456, 1973; and Wigler *et al., Proc Natl Acad Sci USA* 76:1373-76, 1979.

Successfully transformed cells, i.e., cells that contain a multicopy expression system of the present invention, can be identified by well known techniques. Cells from individual colonies can be harvested, lysed and their DNA content examined for the presence of the rDNA using a method such as that described by Southern, *J Mol Biol,* 98:503, 1975, or Berent *et al., Biotech.* 3:208, 1985. Alternatively, copending application U.S. Serial No. 08/642,541 discloses a rapid screening method to identify transformants which express high levels of recombinant methioninase based on a color cast of colonies formed on solid media.

Recombinant methioninase is produced at commercially significant levels using a host transformed with one or more of the multicopy methioninase expression systems of the present invention. Such a transformed host will express recombinant methioninase at a level from about 40-75% of total cellular protein. The protein may be purified if desired.

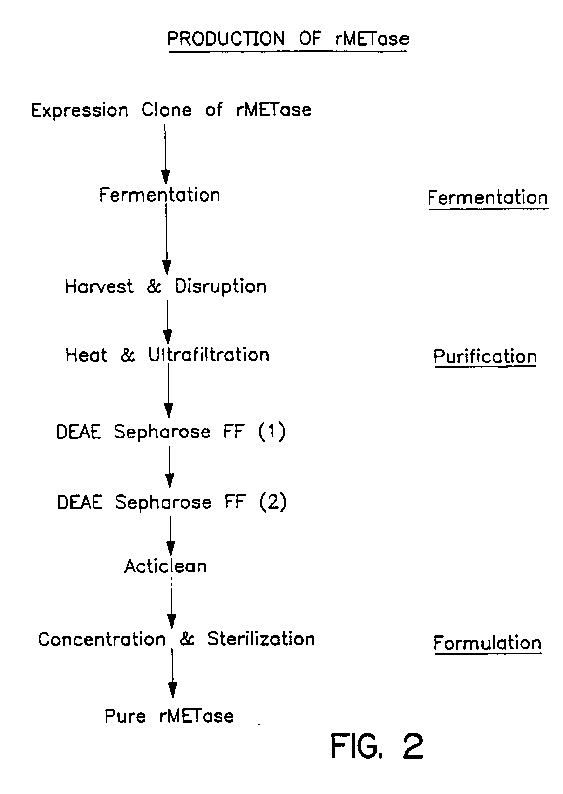

A preferred method for purification is shown in Figure 2 and comprises the steps of:
a) heating an extract of a transformed cell that contains methioninase in aqueous buffers from about 40-60°C for about 1-10 min., preferably 50°C for 1 min.;
b) centrifuging the heated extract from about 10K to 20K rpm in a GS-3 rotor (Sorvall, Du Pont) for about 15 min. to 1 hour, preferably at about 13K rpm for about 30 min. at 4°C;
c) ultrafiltering the supernatant using a filter of about 50K to 100K pore size, preferably a Millipore Pre/Scale:TFF PLHK 100 K 2.5 ft² cartridge using a 10 mM potassium phosphate buffer (pH 8.3);
d) performing DEAE ion exchange chromatography in low ionic strength (from about 10-50 mM) KCl in a 10-20 mM potassium phosphate buffer at about pH 7.0-7.6, and collecting fractions containing methioninase eluted in a 40-200 mM KCl gradient, preferably using DEAE-Sepharose FF column;
e) performing a second DEAE ion exchange chromatography in medium ionic strength (50-100 mM) KCl in a 10-20 mM potassium phosphate buffer at about pH 8.0-8.6, and collecting fractions containing methioninase eluted in a phosphate buffer (pH 8.3) eluted in 100-200 mM KCl, preferably using DEAE-Sepharose FF column; and
f) contacting said fractions collected in step (e) with a chromatography medium capable of absorbing endotoxin, and collecting the eluant, thereby removing endotoxin from said eluant to form endotoxin-free methioninase having at least 20 units methioninase activity per milligram protein and from 1-100 ng of endotoxin per mg protein, preferably using an Acticlean® Etox column.

The cell extract is prepared from a host cell that has been altered to contain one of the multicopy methioninase expression systems of the present invention. For bacterial cell extracts, the extracts are generally prepared by first harvesting and washing bacterial cell cultures to form a cell paste/pellet, depending upon whether harvesting is by centrifugation or by hollow fiber filtration, which methods are generally well known.

The cells are then disrupted using conventional means. Preferably the cells are disrupted using a homogenizer, such as a cavitator-type homogenizer, for example, a Microfluidics Corp. Model #HC8000.

The resulting suspension is heated to precipitate selective proteins and other insoluble materials. Typical heating conditions are from about 45-60°C for 1-10 minutes. Preferred is a heating step of 50°C for 1 minute.

The heated extract is centrifuged to remove debris, and the supernatant is filtered and applied to DEAE ion-exchange chromatography medium in two steps as described above. Preferred adsorption and elution conditions are described in the Examples. Any of a variety of DEAE ion exchange column chromatography media can be used in these steps, and the choice of media is not to be construed as limiting. Commercial sources include Pharmacia Fine Chemicals, BioRad, and Sigma.

Thereafter, endotoxin is removed to produce a protein having acceptable levels of endotoxin as recited earlier. The endotoxin removal step can be carried out in any of a variety of well known means, including contacting the protein in solution with a chromatographic medium capable of adsorbing endotoxin, and eluting the endotoxin-free protein. The preferred commercial reagent for use in removing endotoxin is Acticlean® Etox.

### Therapeutic Compositions

The substantially isolated recombinant methioninase produced using a host transformed with one of the multicopy methioninase expression systems of the present invention may be formulated into therapeutic compositions.

These compositions will preferably contain recombinant methioninase that has a specific activity of about 10 to 50 units (U) per mg protein, typically about 16 tp 24 U/mg.

For inclusion in such compositions, the methioninase is preferably substantially purified. By substantially purified is meant that the enzyme is at least 90% pure by weight, preferably at least 95% pure, and more preferably at least 99% pure, or essentially homogeneous. Homogeneity can be affirmed by polyacrylamide gel electrophoresis (PAGE or SDS-PAGE), to provide only a single detectable band.

The recombinant methioninase should be substantially free of endotoxins, such as bacterial lipopolysaccharides, due to the undesirable side effects associated with endotoxins when physiologically contacted in a mammal, as by i.v. or i.p. administration. By substantially free is meant less than about 10 nanograms (ng) endotoxin per milligram (mg) recombinant methioninase protein, preferably less than 1 ng/mg and more preferably less than 0.1 ng/mg.

The therapeutic compositions may further comprise a physiologically tolerable carrier. As used herein, the terms "pharmaceutically acceptable", "physiologically tolerable" and grammatical variations, both referring to compositions, carriers, diluents and reagents that the materials are capable of administration to or upon a mammal or human without the production of undesirable physiological effects such as nausea, dizziness, gastric upset and the like.

The preparation of a pharmacological composition that contains active ingredients dissolved or dispersed therein is well understood in the art. Typically such compositions are prepared as sterile injectables either as liquid solutions or suspensions, aqueous or non-aqueous, however, solid forms suitable for solution, or suspensions, in liquid prior to use can also be prepared. The preparation can also be emulsified. In addition, a therapeutic amount of recombinant methioninase can be present in a ointment or on a diffusible patch, such as a bandage, as to afford local delivery of the agent.

The active ingredient can be mixed with excipients which are pharmaceutically acceptable and compatible with the active ingredient and in amounts suitable for use in the therapeutic methods described herein. Suitable excipients are, for example, water, saline, dextrose, glycerol, or the like and combinations thereof. In addition, if desired, the composition can contain minor amounts of auxiliary substances such as wetting or emulsifying agents, pH buffering agents and the like which enhance the effectiveness of the active ingredient.

The methioninase may be supplied as a pharmaceutically acceptable salts such as acid addition salts (formed with the free amino groups of the polypeptide) that are formed with inorganic acids such as, for example, hydrochloric or phosphoric acids, or such organic acids as acetic, tartaric, mandelic and the like. Salts formed with the free carboxyl groups can also be derived from inorganic bases such as, for example, sodium, potassium, ammonium, calcium or ferric hydroxides, and such organic bases as isopropylamine, trimethylamine, 2-ethylamino ethanol, histidine, procaine and the like.

Physiologically tolerable carriers are well known in the art. Exemplary of liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, propylene glycol, polyethylene glycol and other solutes.

Liquid compositions can also contain liquid phases in addition to and to the exclusion of water, as described herein. Exemplary of such additional liquid phases are glycerin, vegetable oils such as cottonseed oil, organic esters such as ethyl oleate, and water-oil emulsions, particularly the liposome compositions described earlier.

A therapeutic composition contains an effective amount of recombinant methioninase, typically an amount of at least 0.1 weight percent of active protein per weight of total therapeutic composition, and preferably is at least about 25 weight percent. A weight percent is a ratio by weight of recombinant methioninase protein to total composition. Thus, for example, 0.1 weight percent is 0.1 grams of recombinant methioninase per 100 grams of total composition.

Controlled delivery of the recombinant methioninase can also be effected which further shields the recombinant methioninase protein from degradation and enhances the serum half-life.

Therapeutic compositions may also be delivery vehicles such as polymers, polymeric vehicles, particulates, latexes, coacervates, ion-exchange resins, liposomes, enteric coatings, mediators, bioadhesives, microcapsules, hydrogels, and the like vehicles. Exemplary drug delivery vehicles including liposomcs are described at least by Tarcha in "Polymers For Controlled Drug Delivery", CRC Press, Boca Raton, 1990.

The recombinant methioninase may be chemically modified, for example by conjugation to a polymer or by otherwise altering the covalent structure without changing the primary amino acid sequence. Preferable polymers are polyalkylene oxides or polysaccharides. Coupling to a polymer increases the serum half-life and decreases the immunogenicity or antigenicity of the resulting compound.

A preferred polymer is polyethylene glycol, particularly MSC-5000 PEG, polyethylene oxide, polypropylene oxide, copolymers of ethylene oxide, and copolymers of propylene oxide. Methods for chemically modifying proteins are well known to the art and can readily be used to modify the recombinant methioninase. See, for example, PCT/US93/11311.

### Administration and Utility

Methioninase can be used in diagnostic and therapeutic methods that have been developed and described elsewhere. See PCT/US93/11311. For example, methioninase is used 1) as an antitumor agent in a variety of modalities, such as by depleting methionine from tumor cells, 2) to induce cell cycle stasis in tumor cells followed by cell synchronization and the use of antimitotic agents, 3) in combination with antimitotic and cell cycle-specific cytotoxic agents, 4) to deplete cellular methionine prior to labeling with methionine, which can be used in tumor diagnosis and localization, and 5) to deplete serum homocysteine to prevent and cure cardiovascular diseases that are mediated by high serum levels of homocysteine. Recombinant methioninase prepared according to the present invention has now been administered to nine patients wherein infusion dosage of up to 20,000 units, infused over ten hours, had no significant side effects and yielded a depletion of methionine for 10 hours following infusion.

The following examples relating to this invention are illustrative and do not limit the invention.

### Example 1

### Isolation of Nucleic Acid Molecules Encoding Methioninase

Genomic DNA of *Pseudomonas putida* AC-1, derived from ATCC8209, was used as template; the primers used were as follows: 
t1:5'-GCCGGTCTGTGGAATAAGCT-3' (Sense)
HindIII
 
t2:5'-CCAGGGTCGACTCCAGCGCC-3' (Antisense)
Sal I
 The PCR reaction condition was as follows: first denaturation at 95°C for 10 minutes, then 5 cycles of denaturation at 94°C for 30 seconds, annealing at 60°C for 30 seconds, and extension at 72°C for 2 minutes; then 25 cycles of denaturation at 94°C for 30 seconds, 60°C for 30 seconds, then extension at 72°C for 1.5 minutes; then final extension at 72°C for 10 minutes. The PCR amplified products are two bands of which the 1365 bp band was collected, and purified as the insert ONCase-1 DNA.

The ONCase-1 DNA was ligated with pT7Blue T-vector (Novagen) at the EcoR V T-cloning site. The pONCase-1 DNA was transformed into DH5-α bacterial cells using standard procedures.

DNA sequencing was performed using T7 DNA polymerase and the dideoxy nucleotide termination reaction. The primer walking method was used. [³⁵S] dATP was used for labeling. Sequencing reactions were analyzed on 6% polyacrylamide wedge or non-wedge gels containing 8M urea. DNA samples were loaded in the order of ACGT. DNA sequences were analyzed by MacVector. The DNA sequence and corresponding amino acid sequence are provided in Figure 1.

### Example 2

### High Expression Clones

The pONCase-1 clone was used as the template, the primers used are as follows: 
t14. 5' - GGAATTCCATATGCACGGCTCCAACAAGC - 3' (Sense)
NdeI
 
t15. 5' -
AGTCATCCTAGGTCACATCATCATCATCATCATGGCACTCGCCTTGAGTGC-
3'
BamHI (Antisense)
 
t18. 5' - AGTCATCCTAGGTCAGGCACTCGCCTTGAGTGC - 3' (Antisense)
BamHI
 The PCR reaction condition was as follows: first denaturation at 95°C for 10 minutes, then 5 cycles of denaturation at 94°C for 1 minute, annealing at 56°C for 1.5 minutes, and extension at 72°C for 2 minutes; then 20 cycles of denaturation at 94°C for 30 seconds, 56°C for 30 seconds, then extension at 72°C for 1.5 minutes; then final extension at 72°C for 10 minutes. Two PCR amplified products, ONCase-2 (1238 bp), ONCase-3 (1220 bp) band were collected and purified.

The DNA of ONCase-2 and ONCase-3 DNA was digested with *Ndel* and *BamHI* and ligated with the pT7.7 vector at the *Ndel* and *BamHI* cloning sites. The pONCase-2 and pONCase-3 DNA sequences were then transformed into BL21 (DE3) bacterial cells using standard procedures.

The positive clones were selected from ampicillin-containing plates. After storage at 4°C for 24 hours, the positive clones which expressed high level of recombinant methioninase had a distinct pink color that allowed their identification and selection. The methioninase expression levels of the positive clones were determined by activity assay. Two high expression clones were selected as the pAC-1 clone which contained ONCase-3 and as the pAC-2 clone which contained ONCase-2.

The tetracycline resistance gene was obtained from pBR322 at the *Aval* and *ClaI* sites. The *AvaI* end was filled into a blunt end, ligated with pAC-1 and digested with the *BamHI* and *Clal,* with the *BamHI* end filled into a blunt end. Positive clones which became pink after storage at 4°C for 24 hours were selected from tetracycline-containing plates. A high expression recombinant methioninase clone, pAC-3, was chosen by activity assay.

The tetracycline-resistance gene was also obtained from pBR322 at the *AvaI* and *HindIII* sites. The *Aval* end was filled into a blunt end, and was ligated with pAC-1 which was digested with the *HindIII* and *Clal,* with the *ClaI* end filled into a blunt end. Positive clones which became pink after storage at 4°C for 24 hours were selected from tetracycline-containing plates. A high expression recombinant methioninase clone pAC-4 was determined by activity assay. The levels of expression obtained from these vectors are shown in Table 1.

| **Table 1** | | | | | |
|---|---|---|---|---|---|
| **rMETase Expression Clones** | | | | | |
| **Clone** | **Vector** | **Antibiotic Resistance** | **Promoter** | **Fusion** | **Expression* (g/1)** |
| pAC-1 | pT7.7 | Amp | T7 | --- | 1.0 |
| pAC-2 | pT7.7 | Amp | T7 | His. Tag | 0.5 |
| pAC-3 | pT7.7 | Tc | T7 | --- | 0.5 |
| pAC-4 | pT7.7 | Tc | T7 | --- | 1.0 |
| *Expression level in shaking flask (TB medium, 37°C, 400 rpm, 36 hours). | | | | | |

### Example 3

### Generation of a Tandem Methioninase Expression System

The plasmid pAC-1 was isolated from the amplified cell bank with QIA prep Spin Miniprep Kit (Quiagen), and then digested with *BamHl* to obtain the host vector containing a single methioninase encoding nucleotide sequence.

*ONCase-3* was digested with the *Ndel,* filled in with Klenow Fill-in Kit (Stratagene), and digested with *BarnHI* as the insert for the second methioninase gene. Ligation was performed with DNA Ligation Kit from Stratagene at higher T4 ligase concentration. See Figure 3. The resulting plasmid was then transformed into competent *E*. *coli* BL21 (DE3) cells according to the instruction manual.

Positive clones were selected from ampicillin-containing plates. After optional storage at 4°C overnight, the clones which expressed high levels of methioninase had a distinct yellow-orange color due to high enrichment of the pyridoxal phosphate-containing methioninase. The clone with the highest indication of methioninase content was confirmed by activity assay. When assayed for activity, all yellow-orange colonies were positive for methioninase and noncolored clones were methioninase negative. The clone with highest methioninase activity pAC-11 was selected and confirmed by *BamHI* and *Ndel* digestion.

pAC-11 provides methioninase production as high as 50% of the total intracellular protein as shown in Table 2.

| **Table 2** | | |
|---|---|---|
| **Expression and Stability of pAC-1 and pAC-11** | | |
| | **pAC-1** | **pAC-11** |
| Specific activity of extract (units/mg): | 3 units/mg | 10 units/mg |
| Expression level (% of total protein): | 15% | 50% |
| Stability | Low | Stable |

### Example 4

### Fermentation Of Recombinant Methioninase Expression Clones

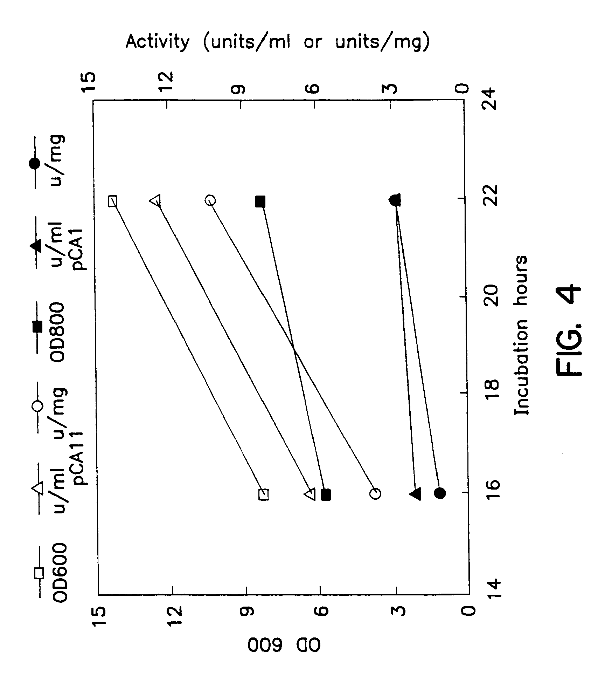

Cells containing pAC-1 and pAC-11 were grown in Terrific Broth medium containing either ampicillin (100µg/ml) or tetracycline (10µg), at 28°C or 37°C with shaking. Table 3 compares the level of methioninase production obtained from these vectors; these results are graphed in Figure 4 to show time course.

| **Table 3** | | | | | |
|---|---|---|---|---|---|
| **Comparisons for PAC-1 and PAC-11 1 Clones** | | | | | |
| **Clone*** | **Growth**** | **OD 600** | | **Activity** | **Specific Activity** |
| | **Hours** | **Total** | **Units/OD** | **Units/ml** | **Units/mg** |
| pAC-1 | 16 | 5.8 | 0.38 | 2.2 | 1.2 |
| | 22 | 8.2 | 0.34 | 2.8 | 2.8 |
| pAC-11 | 16 | 8.2 | 0.78 | 6.4 | 3.8 |
| | 22 | 14.2 | 0.88 | 12.4 | 10.2 |

| | | | | | |
|---|---|---|---|---|---|
| * pAC-1 clone contains one methioninase gene. pAC-11 clone contains two methioninase genes. ** Bacteria growth in 1XTB 800 ml, at 28°C. | | | | | |

### Example 5

### Purification of Recombinant Methioninase

An outline of the purification method is provided in Figure 2.

### (1) Pre-column treatment of the sample

The bacteria were harvested by centrifugation at 800 x g at 4°C for 10 min. The bacterial pellet is then suspended in extraction solution (20 mM potassium phosphate pH 9.0, 10 µM pyridoxal phosphate and 0.01 % β- mercaptoethanol) and disrupted with a cavitator -type homogenizer (Microfluidics Corp. model # HC 8000). Heat treatment of the homogenate is then carried out at 50°C for one minute. The suspension is centrifuged with an automatic refrigerated centrifuge (SORVALL Superspeed RC 2-B) at 4°C at 13 k rpm for 30 min. The supernatant is then collected. This step is followed by ultrafiltration by a Millipore Prep / Scale - TFF PLHK 100k 2.5 ft² cartridge with buffer (10 mM potassium phosphate pH 8.3). The pH is adjusted to 7.2 by ultrafiltration.

### (2) Chromatographic conditions

### The first column: DEAE Sepharose FF

Column: XK 100/60, Height: 32 cm, Volume: 2.5 L
Solution: [A] 40 mM potassium chloride, 10 mM potassium phosphate (pH7.2) containing 10 µM pyridoxal phosphate and 0.01% β - mercaptoethanol. [B] 200 mM potassium chloride, 10 mM potassium phosphate (pH7.2) containing 10 µM pyridoxal phosphate and 0.01 % β-mercaptoethanol.
Flow Rate: 5 ml/min.
Sample: About 100-200 g of total protein (10-20 mg/ml) are applied on the first column.
Gradient: [1] Pre-wash with solution A approximately 10 volumes until the OD₂₈₀ drops below 0.1.
   [2] Gradient: Solution B from 20% - 100%.
Fractions: Elution fractions of 200 ml are collected. The fractions containing methioninase are identified by activity assay and pooled.

### The second column: DEAE Sepharose FF

Column: XK 50/30, Height: 25 cm, Volume: 500 ml
Solution: [A] 100 mM potassium chloride, 10 mM potassium phosphate (pH 8.3) containing 10 µM pyridoxal phosphate and 0.01% β - mercaptoethanol. [B] 200 mM potassium chloride, 10 mM potassium phosphate (pH 8.3) containing 10µM pyridoxal phosphate and 0.01% β - mercaptoethanol.
Flow Rate: 5 ml/min.
Sample: Approximately 10-20 g of total protein (2-4 mg/ml), after dialysis in 100 mM potassium chloride, 10 mM potassium phosphate (pH 8.3) containing 10 µM pyridoxal phosphate for 24 hours, are applied on the second column.
Gradient: [1] Pre-wash with solution A approximately 5 volumes until the OD₂₈₀ drops below 0.05.
   [2] Gradient: Solution B from 0% - 60%.
Fractions: Elution fractions of 200 ml are collected. The fractions containing methioninase are identified by the activity assay and pooled.

### The third column: Sephacryl S-200 HR

Column: HiPrep 26/60, volume 320 ml.
Solution: 0.15 M sodium chloride in 10 mM sodium phosphate (pH7.2)
Flow Rate: 1.2 ml/ min.
Sample: Approximately 10 ml concentrated sample. (after dialysis in 0.15 M sodium chloride, 10 mM sodium phosphate (pH7.2) for 12 hours), are applied to the third column.
Fractions: Elution fractions of 20 ml containing methioninase, which are identified by yellow color and activity assay, are collected.

### The fourth column: Acticlean® Etox

Purified methioninase (10-20 mg protein / ml) in a volume of 100-200 ml is applied on a 500 ml Acticlean® Etox column, and eluted with elution buffer (0.15 M sodium chloride in 10 mM sodium phosphate pH7.2) in order to eliminate endotoxin. Acticlean® Etox is reusable and can be cleaned with 1 M sodium hydroxide and can be autoclaved.

### Concentration of the final eluant

The final eluant is concentrate with 30 K Amicon Centriprep Concentrators. The formulation for purified methioninase is 0.15 M sodium chloride, 10 mM sodium phosphate, pH7.2.

### Purification of Methioninase Histidine: Chromatography on Ni⁺⁺ Sepharose column

The cell homogenate, after pre-column treatment, is suspended in binding buffer (5 mM imidazole, 0.5 M NaCl, 20 mM Tris, HCl, pH7.9). The column is then washed with 10 volumes of binding buffer followed by washes with 6 volumes of wash buffer (60 mM imidazole, 0.5 M sodium chloride, 20 mM Tris, HCl, pH7.9). Elution occurs after 6 volumes of elution buffer (1 M imidazole, 0.5 M NaCl, 20 mM Tris. HC pH7.9) have been run through the column. The fractions containing methioninase, identified by yellow color, are collected.

## Claims

1. An expression vector capable of high level production of methioninase, comprising two or more tandem copies of a nucleotide sequence encoding methioninase operably linked to a promoter
wherein said nucleotide sequence is selected from the group consisting of:
a) a nucleotide sequence that encodes a protein having the amino acid sequence shown in Figure 1,
b) a nucleotide sequence as shown as encoding protein in Figure 1, and
c) a nucleotide sequence that encodes a methioninase which is encoded by a nucleotide sequence that hybridizes to the complementary strand of a nucleotide sequence encoding protein as shown in Figure 1 under high stringency conditions.

2. The expression vector of claim 1, wherein said nucleotide sequence is that shown as encoding protein in Figure 1.

3. The expression vector of claim 1, wherein said nucleotide sequence encodes a protein with the amino acid sequence shown in Figure 1 and comprises the corresponding nucleotide sequence of Figure 1 modified to contain one or more *E. coli* favored codons.

4. The expression vector of claim 1, wherein said promoter is a T7 RNA polymerase promoter.

5. Cells modified to contain the expression vector of any of claims 1 to 4.

6. The cells of claim 5 which are *E*. *coli.*

7. The cells of claim 6, which are *E*. *coli* BL21(DE3).

8. A method for producing methioninase, which method comprises culturing the cells of any of claims 5 to 7 under conditions wherein said methioninase is produced and optionally recovering the methioninase from the culture.

## Patentansprüche

1. Expressionsvektor, der Methioninase in einer hohen Konzentration erzeugen kann, welcher zwei oder mehr Tandemkopien einer Nukleotidsequenz umfasst, die Methioninase kodiert, und die funktionell mit einem Promotor verknüpft ist, wobei die Nukleotidsequenz aus der Gruppe bestehend aus
a) einer Nukleotidsequenz, die ein Protein kodiert, das die in der Figur 1 gezeigte Aminosäuresequenz aufweist,
b) einer Nukleotidsequenz, die als Protein-kodierend in der Figur 1 gezeigt ist, und
c) einer Nukleotidsequenz, die eine Methioninase kodiert, die durch eine Nukleotidsequenz kodiert ist, die an den komplementären Strang einer Nukleotidsequenz, die ein Protein kodiert, gemäß der Fig. 1, unter hochstringenten Bedingungen hybridisiert,
ausgewählt ist.

2. Expressionsvektor nach Anspruch 1, bei dem die Nukleotidsequenz diejenige ist, die als Protein-kodierend in der Figur 1 gezeigt ist.

3. Expressionsvektor nach Anspruch 1, bei dem die Nukleotidsequenz ein Protein kodiert, das die in der Figur 1 gezeigte Aminosäuresequenz aufweist, und die entsprechende Nukleotidsequenz von Figur 1 umfasst, die so modifiziert ist, dass sie ein oder mehrere von E. coli bevorzugte(s) Kodon(s) enthält.

4. Expressionsvektor nach Anspruch 1, bei dem der Promotor der T7 RNA-Polymerasepromotor ist.

5. Zellen, die so modifiziert sind, dass sie den Expressionsvektor nach einem der Ansprüche 1 bis 4 enthalten.

6. Zellen nach Anspruch 5, bei denen es sich um E. coli handelt.

7. Zellen nach Anspruch 6, bei denen es sich um E. coli BL21 (DE3) handelt.

8. Verfahren zur Herstellung von Methioninase, wobei das Verfahren das Kultivieren der Zellen nach einem der Ansprüche 5 bis 7 unter Bedingungen, bei denen die Methioninase erzeugt wird, und gegebenenfalls Gewinnen der Methioninase aus der Kultur umfasst.

## Revendications

1. Vecteur d'expression capable d'une production de méthioninase à un niveau élevé, comprenant deux ou plusieurs copies en tandem d'une séquence nucléotidique codant pour une méthioninase liée de manière fonctionnelle à un promoteur
dans lequel ladite séquence nucléotidique est choisie dans le groupe constitué de :
(a) une séquence nucléotidique qui code pour une protéine ayant la séquence d'acides aminés présentée à la figure 1,
(b) une séquence nucléotidique telle que montrée comme codant pour une protéine à la figure 1, et
(c) une séquence nucléotidique qui code pour une méthioninase qui est codée par une séquence nucléotidique qui s'hybride avec le brin complémentaire d'une séquence nucléotidique codant pour une protéine telle que montrée à la figure 1 dans des conditions de stringence élevée.

2. Vecteur d'expression de la revendication 1, dans lequel ladite séquence nucléotidique est celle montrée comme codant pour une protéine à la figure 1.

3. Vecteur d'expression de la revendication 1, dans lequel ladite séquence nucléotidique code pour une protéine avec la séquence d'acides aminés montrée à la figure 1 et comprend la séquence nucléotidique correspondante de la figure 1 modifiée pour contenir un ou plusieurs codons préférés de *E. coli.*

4. Vecteur d'expression de la revendication 1, dans lequel ledit promoteur est un promoteur de l'ARN polymérase T7.

5. Cellules modifiées pour contenir le vecteur d'expression de l'une quelconque des revendications 1 à 4.

6. Cellules de la revendication 5 qui sont *E. coli.*

7. Cellules de la revendication 6, qui sont *E. coli* BL21 (DE3).

8. Méthode de production de méthioninase, laquelle méthode comprend la culture de cellules de l'une quelconque des revendications 5 à 7 dans des conditions dans lesquelles ladite méthioninase est produite et éventuellement de récupération de la méthioninase à partir de la culture.
